(19) Europäisches Patentamt
European Patent Office
Office européen des brevets

(11) EP 4 714 931 A1

(12) EUROPEAN PATENT APPLICATION
published in accordance with Art. 153(4) EPC

(43) Date of publication:
**25.03.2026 Bulletin 2026/13**

(21) Application number: **24713991.8**

(22) Date of filing: **27.02.2024**

(51) International Patent Classification (IPC):
***C05F 17/00*** (2020.01) ***C05G 5/20*** (2020.01)
***A01N 37/44*** (2006.01) ***A01N 37/46*** (2006.01)
***A01P 21/00*** (2006.01) ***A01N 33/00*** (2006.01)

(52) Cooperative Patent Classification (CPC):
(C-Sets available)
**A01N 37/46; A01P 21/00; C05F 11/10; C05G 5/20**
(Cont.)

(86) International application number:
**PCT/ES2024/070110**

(87) International publication number:
**WO 2024/236206 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.05.2023 ES 202330382**

(71) Applicant: **Comercial Química Massó S.A.**
**08029 Barcelona (ES)**

(72) Inventors:
- **PÉREZ LEBEÑA, Eduardo**
**08029 Barcelona Barcelona (ES)**
- **VIRGILI OLIVÉ, Albert**
**08029 Barcelona Barcelona (ES)**

(74) Representative: **Ungria López, Javier**
**Avda. Ramón y Cajal, 78**
**28043 Madrid (ES)**

(54) **TERNARY INCLUSION COMPLEX OF GLYCINE-BETAINE, MONOSODIUM GLUTAMATE AND PROLINE, METHOD FOR OBTAINING SAME AND USE THEREOF**

(57) The invention relates to a method for obtaining a ternary inclusion complex of glycine-betaine, monosodium glutamate and proline, in aqueous solution, which comprises: (a) mixing between 2.5% and 6% w/v of monosodium glutamate and between 4% and 7% w/v of proline in water and stirring; (b) adding between 30 and 45% w/v of glycine-betaine and adding water until reaching 100% w/v; heating to a temperature between 30 and 50°C, stirring and applying ultrasound until achieving complete dissolution of the proline, monosodium glutamate and glycine-betaine in water, followed by obtaining the inclusion complex in aqueous solution. Another object of the invention is the inclusion complex obtained from said method and its use as a fertilising agent, biostimulant, or as an agent to mitigate abiotic stress.

EP 4 714 931 A1

(52) Cooperative Patent Classification (CPC): (Cont.)

C-Sets
**A01N 37/46, A01N 37/46, A01N 43/36**

## Description

### TECHNICAL SECTOR

The present invention falls within the general scope of Chemistry, more specifically within Organic Chemistry, and it is applicable in the agricultural, forestry and/or gardening sectors. More particularly, it falls within the sector of compositions acting as regulators of abiotic stress in plants in general, in agricultural crops, forestry and/or gardening.

### BACKGROUND OF THE INVENTION

Several environmental factors negatively affect the growth and development of plants and the final yield of crops. Drought, salinity, nutrient imbalances (including mineral toxicities and deficiencies) and extreme temperatures are some of the major environmental constraints to crop productivity worldwide. These factors induce the so-called abiotic stress.

It is estimated that less than 10% of the world's arable land can be free from environmental disturbances, where the most widespread is drought and salinity. For example, up to 45% of the world's agricultural land is subject to continuous or frequent droughts, where 38% of the world's human population resides, and the global surface area affected by salinity is more than 3 million $km^2$, approximately 6% of the total surface area. Furthermore, 19.5% of irrigated agricultural land is considered saline and each year 2 million hectares (approximately 1%) of the world's agricultural land deteriorates due to salinity, which leads to a reduction or absence of crop productivity. Low rainfall, high surface evaporation, the weathering of native rocks, saline water irrigation and poor cultural practices are some of the main factors contributing to increased salinity. Secondary salinisation exacerbates the problem, since agricultural lands, which were previously productive, are becoming unsuitable for cultivation due to the poor quality of irrigation water.

Climate change is on the horizon, and its implications will have a significant impact on crop productivity and, accordingly, on food and nutritional security. The physiological and metabolic activities of crop plants are radically altered not only by the increase in daytime temperatures, but also by high night temperatures. All important food crops, such as cereals and legumes, are prone to high temperature stress (HTS), causing an overall reduction in yield. In addition to the loss of yield derived from the HTS, several physiological changes occur such as a decrease in photosynthetic efficiency and the formation of reactive oxygen species (ROS) leading to oxidative stress particularly in chloroplasts, with a consequence of damage to the membrane, and the onset of leaf senescence. To generate climate-resistant crops, it is necessary to understand the underlying physiological and biochemical mechanisms associated with HTS.

High temperature stress causes changes in plant growth, development, leaf senescence, the discolouration thereof, chlorophyll degradation and the decrease in photosynthetic efficiency. The integrity of the chloroplasts collapses, which leads to programmed cell death. Under these conditions, metabolic reprogramming occurs such as chlorophyll breakdown, ROS production and alterations in carbon metabolism. This alteration in metabolic programming is perhaps essential during stress acclimatisation. Chloroplasts play a crucial role in inducing the expression of nuclear heat-responsive genes under the response to HTS, and light intensity inhibits chlorophyll synthesis. Heat stress causes a reduction in nitrogen concentrations in leaves, damage to proteins and oxidative stress, limiting plant growth and productivity. Temperature stress reduces chlorophyll biosynthesis due to the decrease in the enzymes that biosynthesise it, such as 5-aminolevulinate dehydratase, which is related to oxidative damage.

The upper plants are sessile and, therefore, cannot escape adverse environmental conditions, which constitute a constant threat throughout their life cycle. Unfavourable conditions for growth, such as extreme temperatures (heat, cold and freezing), drought (poor rainfall and dry winds) and soil contamination with high concentrations of salt, are considered the main abiotic environmental stressors that can not only limit plant growth and development, but also determine the geographical distribution of plant species and directly affect agronomic yield. The early effects of high salinity and drought on plant metabolism are relatively similar since they both restrict water availability to plant cells and impose osmotic stress that can lead to loss of turgor.

To deal with this, plants react with stomatal closure, which inhibits $CO_2$ assimilation and, therefore, triggers a chain of events that includes the accumulation of reducing equivalents, the reduction of plastidial and mitochondrial electron transport chains and, as a result, increasing the production of reactive oxygen species ROS which, in turn, damage proteins, lipids and nucleic acids. Prolonged saline stress also induces hyperionic stress and secondary deficiencies of $K^+$ and $NO_3^-$. In field conditions, this may even be the predominant factor affecting plant yield. Plants have developed different strategies to minimise the adverse effects of abiotic stress conditions and several of them are related to amino acid metabolism. For example, osmotic adjustment is achieved through the accumulation of compatible osmolytes that do not interfere with plant metabolism even at high concentrations and can also act as ROS scavengers.

Lastly, the characteristics that must be achieved by an abiotic stress regulator can be summarised as follows:

- acts as a mediator of osmotic adjustment;
- stabilises proteins and cell membranes;

- induces genes related to osmotic stress;
- is a readily available source of carbon and nitrogen in cellular rehydration;
- is a source of support for oxidative phosphorylation;
- generates ATP during post-stress recovery;
- helps control cytosol acidosis and maintain the NADH/NAD+ ratio at values compatible with metabolism;
- helps in the detoxification of excess ammonium cation $NH_4^+$; and
- helps cells overcome oxidative stress.

## DESCRIPTION OF THE INVENTION

A first object of the invention is a method for obtaining a ternary inclusion complex composed of glycine-betaine, monosodium glutamate and proline, in aqueous solution, which comprises the following steps:

a) mixing between 2.5% and 6% w/v, and more preferably between 2.5% and 4% w/v, of monosodium glutamate and 4% to 7% w/v, and more preferably between 5% and 6% w/v, of proline in water and stirring until obtaining a homogeneous dispersed mixture. Particularly, this step can be carried out by initially adding monosodium glutamate to water, stirring and, subsequently, adding proline to the previous mixture. Likewise, in a preferred embodiment of the invention, this mixing step can be assisted by the application of ultrasound, after adding monosodium glutamate and proline to water;
b) adding between 30 and 45% w/v, and more preferably between 40% and 45% w/v, of glycine-betaine with respect to the total dispersed mixture obtained in the previous step and adding water until reaching 100% w/v;
c) heating to a temperature of between 30 and 50°C, stirring preferably at a stirring rate of between 300 and 1000 rpm and applying ultrasound with a frequency preferably between 15 and 25 kHz, and more preferably between 20 and 25 HZ, until complete dissolution of all components and formation of the inclusion complex of proline, monosodium glutamate and glycine-betaine in aqueous solution. Particularly, this step can be carried out for a time between 5 and 15 minutes; and
d) preferably, allowing the aqueous solution to cool until it reaches a temperature below 20°C, preferably between 10°C and 20°C.

A second object of the invention is an inclusion complex of proline, monosodium glutamate and glycine-betaine in aqueous solution, which comprises, by weight with respect to the total volume:

a) between 2.5 and 6% w/v, and more preferably between 2.5% and 4% w/v, of monosodium glutamate and between 4 and 7% w/v, and more preferably between 5% and 6% w/v, of proline,
b) between 30 and 45% w/v, and more preferably between 40% and 45% w/v, of glycine-betaine, and
c) water until reaching 100% w/v of the aqueous solution,

and wherein said inclusion complex of proline, monosodium glutamate and glycine-betaine in aqueous solution does not contain any chemical surfactant or emulsifier.

Particularly, the density of the claimed inclusion complex in aqueous solution can vary between 1.08 and 1.12 kg/l (at 25°C), depending on the amount of monosodium glutamate, proline and glycine-betaine that are added to the process (the lower the percentage, the lower the density of the final solution).

One of the main advantages that the inclusion complex of proline, monosodium glutamate and glycine-betaine in aqueous solution, the object of the invention, has compared to other products described in the prior art is the surprising improvement it exhibits in terms of the solubility of its active ingredients, monosodium glutamate, proline and glycine-betaine, in water.

Additionally, it has the advantage that it is a harmless product, based on natural, safe and biodegradable substances, which are even human food grade. It is important to note that the components of the claimed inclusion complex of proline, monosodium glutamate and glycine-betaine in aqueous solution are generally recognised as safe (GRAS), thus favouring its use in the treatment of crops, since it does not imply additional safety measures on the part of the applicators, a very important factor in agriculture. Therefore, it is an environmentally friendly product that does not damage ecosystems, since it does not include toxic substances. In particular, it makes it possible to improve the bioavailability of proline, eliminating the presence of other chemicals essential for its dissolution, as surfactants or emulsifiers, currently used in the agricultural sector.

The use of the claimed inclusion complex as a fertilising agent is another object of the invention. In the context of the present invention, fertiliser is understood to be a composition that comprises the essential nutrients for the growth and development of a plant. The inclusion complex object of the invention has a fertilising function due to the presence of amino acids or pro-amino acids in the composition itself. In this regard, it performs the fertilising function *per se,* preventing the

plant from having to produce nutrients from the nitrogen fixed through its roots, with the consequent economic savings that this entails. Additionally, there are numerous additional benefits associated with the presence of amino acids in the composition, acting on various aspects such as resistance to stress, photosynthesis, pollination, the activation of phytohormones and other growth substances, the balance of the flora in the soil and the absorption and translocation system of microelements at the plant level.

Additionally, the use of the claimed inclusion complex as a biostimulating agent is also an object of the invention. In the context of the present invention, biostimulant is understood to be a substance that alters plant processes, improving its yield. In particular, the inclusion complex object of the invention has the ability to stimulate the growth system of the plant under abiotic stress conditions, due to high temperatures, drought or excessive salinity in the land.

Lastly, the use of the claimed inclusion complex as an agent to mitigate abiotic stress, induced by lack of irrigation water, high temperatures or excessive salinity, is another object of the invention.

## BRIEF DESCRIPTION OF THE FIGURES

Along with this description, a number of figures are filed wherein the results of the experimental assays carried out to test the effectiveness of the invention are shown in an illustrative and non-limiting manner. In particular:

- Figure 1 shows a photograph of the lettuce *Lollo rosso* treated as described in example 3, 64 days after treatment, along with a size scale in centimetres;
- Figure 2 shows the survival percentage of the lettuce being assayed as described in example 3;
- Figure 3 shows the mean diameter (in centimetres) of the lettuce being assayed as described in example 3;
- Figure 4 shows the mean weight of the lettuce (in grams) being assayed as described in example 3;
- Figure 5 shows the root length (in centimetres) of the lettuce being assayed as described in example 3.
- Figure 6 shows the root weight (in grams) of the lettuce being assayed as described in example 3.

## DETAILED DESCRIPTION OF THE INVENTION

As described earlier, the present invention offers a novel process for increasing the solubilisation of proline in water in the presence of other solutes, not having been described to date in the technical literature. The claimed novel inclusion complex of proline, monosodium glutamate and glycine-betaine in aqueous solution successfully increases the solubility of proline in water up to 4 times and is especially suitable for use in the preparation of natural and bioavailable fertiliser, biostimulant and abiotic anti-stress compositions.

In particular, the developed method makes it possible to obtain a complex of monosodium glutamate/proline/-glycine-betaine through a mixing process at medium/low temperature with ultrasound application. The inclusion complex of proline, monosodium glutamate and glycine-betaine in aqueous solution obtained through said process can be subsequently diluted in water to a percentage that allows its use in agriculture, forestry and/or gardening. In particular, it has been shown that, surprisingly, the amino acids of the claimed inclusion complex remain soluble after dilution in large quantities of water, without precipitating or without forming an isolated powder on the free surface of the liquid. This is due to the phenomenon described above with regard to the bioavailability of the inclusion complex of proline, monosodium glutamate and glycine-betaine in aqueous solution, which allows for a greater fertilising, biostimulant and abiotic anti-stress effect when applied to crops. In particular, it has been demonstrated that glycine-betaine form an inclusion complex in aqueous solution that increases the solubility of proline in water at least 4-fold. In this regard, the application of the claimed inclusion complex diluted in water is another object of the invention. This way, for example, 1 litre of the claimed inclusion complex in aqueous solution may be used diluted in 50, 100, 200, 300, 400 or 500 litres of water, without losing its solubility.

In the context of the present invention, the formation of the complex occurs as a result of the loss of the proton by both carboxylic groups of the amino acids, which move, in the case of proline, to the amino group of glycine-betaine.

The effectiveness of the product object of the present invention, together with its high bioavailability, makes it a truly advantageous product compared to fertilisers, biostimulants and/or abiotic anti-stress agents known on the market, which barely dissolve 2% of proline in water in the presence of other solutes. With this, the claimed inclusion complex of proline, monosodium glutamate and glycine-betaine in aqueous solution is constituted by effective natural components, thus avoiding the use of chemicals responsible for toxic effects on the environment.

As described above, it is noted that, given the chemical composition of the claimed inclusion complex of proline, monosodium glutamate and glycine-betaine in aqueous solution, the solubility of proline has increased. In fact, it has been shown that the solubility of proline increases with the concentration of glycine-betaine, present between 30% and 45% by weight, thus facilitating the formation of the inclusion complex of proline, monosodium glutamate and glycine-betaine in aqueous solution between both amino acids due to weak or short-range molecular interactions between the molecules of both compounds in water. In particular, these interactions consist of hydrogen bonds between the two amino acids

described, i.e., the soluble and the least soluble in water. A hydrogen bond is an electrostatic force that provides great stability, but it is weaker than a covalent bond or an ionic bond. In the appearance of these bonds, the action of ultrasound that is applied during the method for obtaining the inclusion complex of proline, monosodium glutamate and glycine-betaine in aqueous solution is essential.

In particular embodiments of the present invention, the aqueous solution of the claimed inclusion complex of proline, monosodium glutamate and glycine-betaine has a pH between 7.5 and 9 and a cationic nature, derived from the presence of the amino group of glycine-betaine and proline, with a great tendency to capture the proton of the carboxylic group of proline and become positively charged, which provides the formed complex with the cationic nature. In preferred embodiments, the pH of the aqueous solution will be between 8 and 9.

A major advantage of the claimed inclusion complex of proline, monosodium glutamate and glycine-betaine in aqueous solution is that it does not require the incorporation of emulsifiers or surfactants to improve its solubility and bioavailability in aqueous solution, unlike other alternative products of the prior art, which are associated with a significant risk of toxicity in crops, as well as in soils intended for agriculture, forestry and gardening in general.

Therefore, this is a very advantageous solution compared to the compounds that currently exist on the market, since with a simple preparation of the inclusion complex of proline, monosodium glutamate and glycine-betaine in aqueous solution, a very stable product is obtained, which is carried in a more elemental way than other alternatives based on oils and emulsifiers, polyelectrolytes, as well as other more complicated encapsulation products, with the consequent cost savings. Although it is known that the stability of solutions can be improved by adding a surfactant agent, for reasons of environmental sustainability, the product object of the invention does not contain them in its composition.

In a preferred embodiment, the fertiliser, biostimulant and abiotic anti-stress composition may additionally comprise at least one additive, preferably a preservative, and more preferably potassium metabisulfite. Preferably, said additive will be present in an amount between 0.1% and 0.5% by weight with respect to the total volume of the aqueous solution. In even more preferred embodiments, the amount of additive (preferably, preservative and more preferably potassium metabisulfite) may be 0.2%, 0.3% or 0.4% by weight with respect to the total volume. Said additive can be added in a last step of the process, after the formation of the inclusion complex of proline, monosodium glutamate and glycine-betaine in aqueous solution.

## PARTICULAR EMBODIMENTS

*Example 1. Method for obtaining and characterising an inclusion complex of proline, monosodium glutamate and glycine-betaine in aqueous solution as claimed*

It starts with the production of the complex between both amino acids, in accordance with the following composition and method:
50 grams of the amino acid proline in solid powder form are added to 575 grams of water, and after 5 minutes of stirring, 25 grams of monosodium glutamate are added and sonication (ultrasound application) is carried out. Subsequently, 450 grams of glycine-betaine are added under stirring at 300 to 800 rpm. It is heated until a temperature of 30° to 50°C is reached and the ultrasound is applied again, leading to the formation of the inclusion complex of proline, monosodium glutamate and glycine-betaine in aqueous solution. Sonication is carried out for periods of 2 minutes each, and for a total time of 10 minutes, not allowing the solution to heat above 50°C. The resulting solution, protected from light, changes colour until it acquires a yellow ochre colour. Subsequently, the already formed inclusion complex of proline, monosodium glutamate and glycine-betaine can be packaged. The pH of the resulting solution cannot be altered to avoid the formation of undesirable precipitates.

*Example 2. Analysis and theoretical determination of the formation of the inclusion complex of proline, monosodium glutamate and glycine-betaine in aqueous solution*

The theoretical verification of the formation of the complex is based on the study of its energy content, which was calculated using the ChemBio3D Ultra 16.0 software (Perkin-Elmer Inc., Waltham, MA, USA) and the Molecular Mechanics calculation method (MM2). Calculations were performed to obtain the energy of the 3D molecular structure, analysing and presenting the energy of the basal state. The lower the final energy, the greater the stability of the molecule. MM2 is a method that is used to determine the geometry, molecular energies, vibrational spectra and enthalpies of formation of molecules in their basal state and it is commonly used to determine the behaviour of large molecules of biological and pharmaceutical importance. It is usually used to determine the geometries of large molecules, such as those of biological and pharmaceutical importance, which are beyond the reach of more intensive methods based on molecular orbitals. For this reason, MM2 is not useful for modelling transition states of chemical processes with a large spectrum of experimental steps.

Calculations were performed to obtain the energy of different conformations of the same 3D complex, analysing

and presenting those with lower formation energy. Different three-dimensional conformations were estimated for the formed complex. The total energy content, expressed in kcal/mol, of each molecule is defined as the sum of the following interactions:

$$E_{Total} = E_{Stretching} + E_{Bending} + E_{Torsion} + E_{Non\text{-}bonded\ interaction}$$

To verify the energy content of the two amino acids separately and of the complex formed between them, their conformational structure and their interrelation were studied through hydrogen bridge formation. The results were the following:

| Compound | Molecular structure | Energy content |
|---|---|---|
| Glycine-betaine | 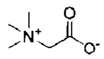 | -74.74 kcal/mol |
| Proline | 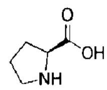 | 6.92 kcal/mol |
| Monosodium glutamate | 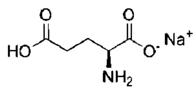 | -124.18 kcal/mol |

A first study of hydrogen bridge formation in the inclusion complex of proline, monosodium glutamate and glycine-betaine in aqueous solution was conducted, obtaining an energy content of -474.94 kcal/mol. In another different conformational structure, an energy content of -275.40 kcal/mol is obtained, as explained below:

| Molecular conformation | Energy content |
|---|---|
| 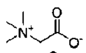 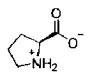 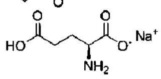 | -474.94 kcal/mol |
| 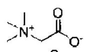 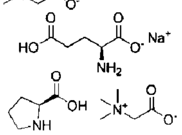 | -275.40 kcal/mol |

From the energy study of the different possibilities of formation of the complex between proline and glycine-betaine through hydrogen bridge formation, it is concluded that the first study is the most energetically favourable and, therefore, the one that happens, since the protonation of the amino group of glycine-betaine and the free amino group of proline occurs through the transfer of protons from the carboxylic groups present in both amino acids.

*Example 3. In vivo test carried out with the complex described in Example 1.*

A summary of the methodology and the results obtained in the *in vivo* tests that have been carried out is provided:

**Test design and objective:**

In this assay, the effectiveness of the product called STRESSLESS LIQUID compared to water stress in lettuce *Lollo rosso,* obtained in accordance with the process described in example 1, is studied. The study was performed in the experimental fields of the company NEVAL and consists of 3 trials, including two trials subjected to water stress (70% irrigation) and a control with 100% irrigation. The trial was treated with STRESSLESS LIQUID in a concentration of 2 ml/l of water. Furthermore, the evolution of this trial was compared with that of a non-stressed control (100% irrigation). Each trial

was made up of 30 pots of lettuce *Lollo rosso,* which were watered twice a week. The assay started after the transplant, when complete irrigations for the 100% control trial and reduced irrigations (70% water compared to the 100% control) began. The first application was made at the time the symptoms began and, the second, after 21 days. Figure 1 shows a photograph of the lettuce *Lollo rosso* 64 days after treatment.

At the end of the assay, some analyses were carried out. The mean weight of the lettuce (g/lettuce), as well as its diameter and the length and weight of the roots were evaluated. Furthermore, the survival percentage was evaluated in each trial, each made up of 30 pots (10 pots per block).

**Location:**

| | |
|---|---|
| Country | SPAIN |
| Province | XILXES |
| Municipality | CASTELLÓN |
| Zone / Plot | INSTALACIONES DE NEVAL |
| GPS data | N/A |

**Methods during the assay:**

| Date | Activity | Interval |
|---|---|---|
| 22/11/2022 | Transplant | 0 |
| 13/12/2022 | Ap. A | 0 DDA |
| 03/01/2023 | Ap. B | 21 DDA |
| 08/03/2023 | Final analyses:<br>· Aerial weight and diameter<br>· Root length and weight<br>· Survival % | 64 DDA |
| Abbreviations: DDA: Days after application; Ap.: Application | | |

**Maintenance during the study:**

| | |
|---|---|
| Irrigation | Twice a week |
| Amount of water received | CONTROL 100: 300 ml/week<br>CONTROL 70, STRESSLESS LIQUID: 210 ml/week |
| Fertilisation | Not performed |
| Plant protection | Not performed |

**Meteorology:** The assay was performed in pots in the field. Meteorological data was obtained from the Vall d'Uixó (Castellón) weather station.

**Conclusions:**

Figure 2 shows the survival percentage of the samples treated with STRESSLESS LIQUID.

Regarding the results relative to the size (diameter) of the lettuce head, control 100 (13.10 cm) and control 70

(11.66 cm) showed significant differences between the two, as shown in figure 3.

Furthermore, an increase in the mean weight per lettuce with STRESSLESS LIQUID was also observed, obtaining a significant and clear difference with the control subjected to stress, as shown in figure 4.

The lettuce treated with STRESSLESS LIQUID also showed greater root growth than control 70%, as shown in figure 5, which indicates a greater tolerance to stress.

Lastly, the root weight of the lettuce treated with STRESSLESS LIQUID showed significant differences with control 70%, obtaining values practically identical to those of the 100% control, as shown in figure 6.

## Claims

1. A method for obtaining a ternary inclusion complex composed of glycine-betaine, monosodium glutamate and proline, in aqueous solution, **characterised in that** it comprises the following steps:

    a) mixing between 2.5% and 6% w/v of monosodium glutamate and between 4% and 7% w/v proline in water and stirring until obtaining a homogeneous dispersed mixture;
    b) adding between 30 and 45% w/v of glycine-betaine with respect to the total dispersed mixture obtained in the previous step and adding water until reaching 100% w/v;
    c) heating to a temperature of between 30 and 50°C, stirring and applying ultrasound until achieving complete dissolution of the proline, monosodium glutamate and glycine-betaine in water, followed by obtaining the inclusion complex of proline, monosodium glutamate and glycine-betaine in aqueous solution.

2. The method, according to claim 1, wherein said method comprises a final additional step that comprises allowing the aqueous solution to cool until it reaches a temperature below 20°C.

3. An inclusion complex of proline, monosodium glutamate and glycine-betaine in aqueous solution, **characterised in that** it comprises, by weight with respect to the total volume:

    a) between 2.5 and 6% w/v of monosodium glutamate,
    b) between 4 and 7% w/v of proline,
    c) between 30 and 45% w/v of glycine-betaine, and
    d) water until reaching 100% w/v of the aqueous solution,

    and wherein said inclusion complex of proline, monosodium glutamate and glycine-betaine in aqueous solution does not contain any chemical surfactant or emulsifier.

4. The inclusion complex, according to claim 3, wherein the density of the inclusion complex in aqueous solution varies between 1.08 kg/l and 1.12 kg/l at 25°C.

5. Use of the inclusion complex, according to claim 3 or 4, as a fertilising agent.

6. Use of the inclusion complex, according to claim 3 or 4, as a biostimulating agent.

7. Use of the inclusion complex, according to claim 3 or 4, as an agent to mitigate abiotic stress.

8. Use of the inclusion complex according to claim 3, in agriculture, forestry and/or gardening, wherein said use comprises the dilution of the inclusion complex in aqueous solution in water prior to its use.

9. A fertiliser, biostimulant and abiotic anti-stress composition **characterised in that** it comprises an inclusion complex according to claim 3 or 4.

10. The composition, according to claim 9, wherein said composition additionally comprises at least one additive consisting of a preservative comprised between 0.1% and 0.5% by weight with respect to the total volume of the aqueous solution.

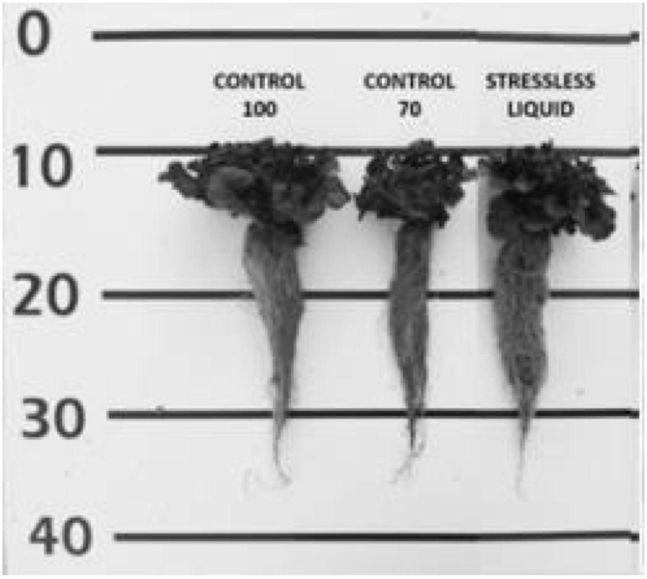
0
10
20
30
40
CONTROL
100
CONTROL
70
STRESSLESS
LIQUID

FIG.1

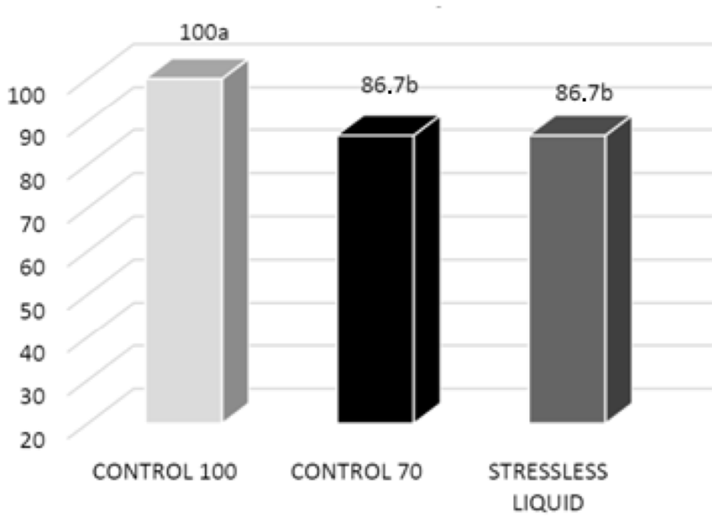
100a
86,7b
86,7b
100
90
80
70
60
50
40
30
20
CONTROL 100
CONTROL 70
STRESSLESS
LIQUID

FIG.2

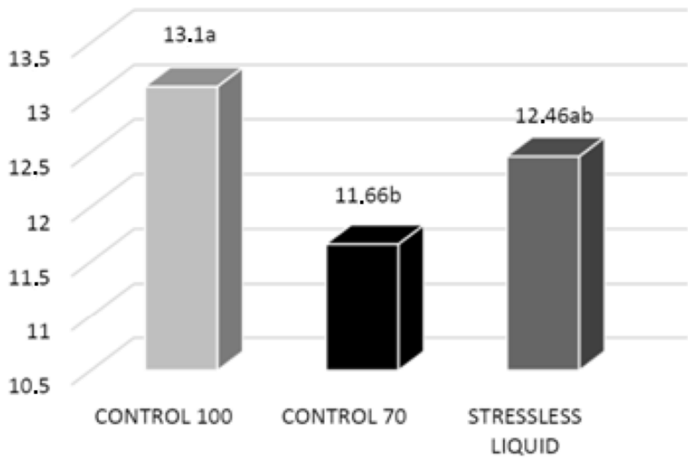
13.5
13
12.5
12
11.5
11
10.5
13.1a
11.66b
12.46ab
CONTROL 100
CONTROL 70
STRESSLESS LIQUID

**FIG.3**

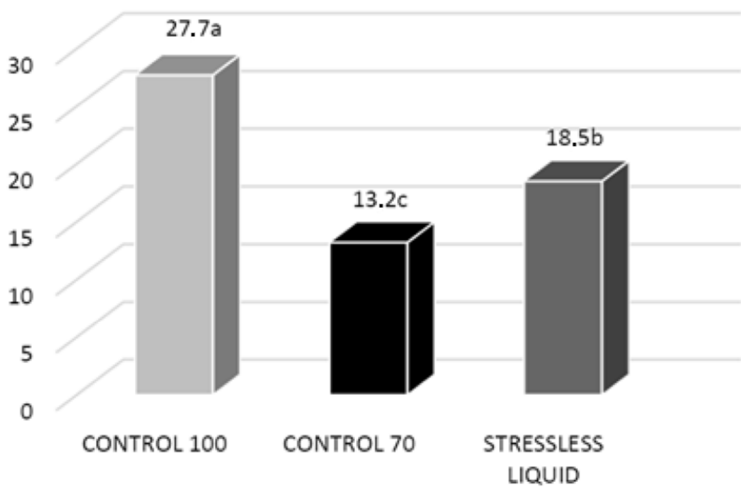
30
25
20
15
10
5
0
27.7a
13.2c
18.5b
CONTROL 100
CONTROL 70
STRESSLESS LIQUID

**FIG.4**

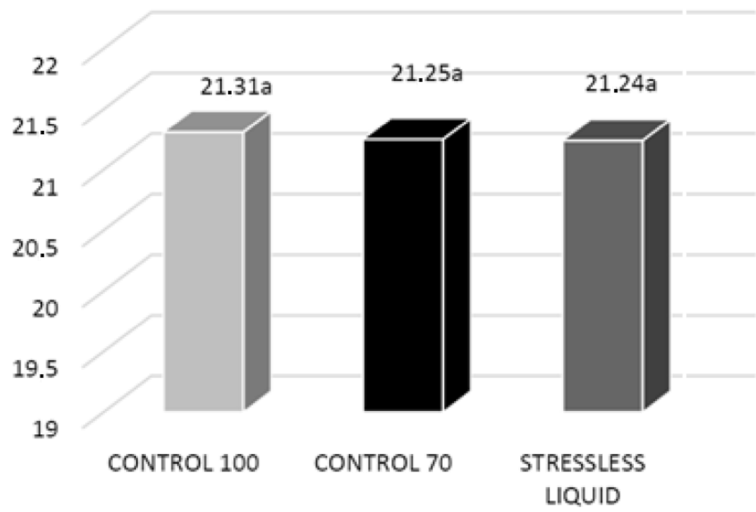
22
21.5
21
20.5
20
19.5
19
21.31a
21.25a
21.24a
CONTROL 100
CONTROL 70
STRESSLESS LIQUID

FIG.5

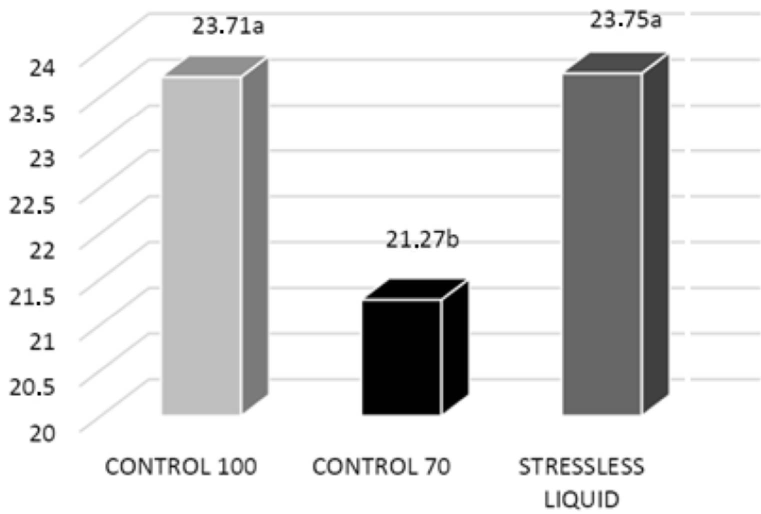
24
23.5
23
22.5
22
21.5
21
20.5
20
23.71a
21.27b
23.75a
CONTROL 100
CONTROL 70
STRESSLESS LIQUID

FIG.6

# INTERNATIONAL SEARCH REPORT

International application No
PCT/ES2024/070110

**A. CLASSIFICATION OF SUBJECT MATTER**
INV. C05F17/00 C05G5/20 A01N37/44 A01N37/46 A01P21/00
A01N33/00
ADD.

According to International Patent Classification (IPC) or to both national classification and IPC

**B. FIELDS SEARCHED**

Minimum documentation searched (classification system followed by classification symbols)
C05F C05G A01N

Documentation searched other than minimum documentation to the extent that such documents are included in the fields searched

Electronic data base consulted during the international search (name of data base and, where practicable, search terms used)

EPO-Internal, WPI Data

**C. DOCUMENTS CONSIDERED TO BE RELEVANT**

| Category* | Citation of document, with indication, where appropriate, of the relevant passages | Relevant to claim No. |
|---|---|---|
| A | WO 2018/187345 A1 (SPOGEN BIOTECH INC [US]) 11 October 2018 (2018-10-11)<br>paragraph [0006]<br>paragraph [0055]<br>paragraph [0059]<br>paragraph [0180]<br>paragraph [0204]<br>paragraph [0383]<br>claims 1,20,21<br>----- | 1-10 |

-/--

[x] Further documents are listed in the continuation of Box C.

[x] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 7 June 2024 | 19/06/2024 |

| Name and mailing address of the ISA/ | Authorized officer |
|---|---|
| European Patent Office, P.B. 5818 Patentlaan 2<br>NL - 2280 HV Rijswijk<br>Tel. (+31-70) 340-2040,<br>Fax: (+31-70) 340-3016 | Rodriguez Fontao, M |

Form PCT/ISA/210 (second sheet) (April 2005)

## INTERNATIONAL SEARCH REPORT

International application No
PCT/ES2024/070110

C(Continuation). DOCUMENTS CONSIDERED TO BE RELEVANT

| Category* | Citation of document, with indication, where appropriate, of the relevant passages | Relevant to claim No. |
|---|---|---|
| A | ALI FARMAN ET AL: "Recent methods of drought stress tolerance in plants", PLANT GROWTH REGULATION, SPRINGER NETHERLANDS, DORDRECHT, vol. 82, no. 3, 22 March 2017 (2017-03-22), pages 363-375, XP036256277, ISSN: 0167-6903, DOI: 10.1007/S10725-017-0267-2 [retrieved on 2017-03-22] *Physiological mechanism of drought stress tolerance Osmolytes production and osmotic stress tolerance in plants*; pages 1,2 | 1-10 |
| A | CN 109 734 539 B (SHENZHEN BATIAN ECOTYPIC ENG) 8 February 2022 (2022-02-08) abstract claims | 1-10 |
| A | CALVO PAMELA ET AL: "Agricultural uses of plant biostimulants", PLANT AND SOIL, SPRINGER INTERNATIONAL PUBLISHING, CHAM, vol. 383, no. 1, 8 May 2014 (2014-05-08), pages 3-41, XP035395225, ISSN: 0032-079X, DOI: 10.1007/S11104-014-2131-8 [retrieved on 2014-05-08] Protein hydrolysates and amino acids; page 23ff | 1-10 |
| A | FR 2 803 176 B1 (SAMABIOL [FR]) 21 November 2003 (2003-11-21) claims | 1-10 |

Form PCT/ISA/210 (continuation of second sheet) (April 2005)

## INTERNATIONAL SEARCH REPORT

Information on patent family members

International application No
PCT/ES2024/070110

| Patent document cited in search report | Publication date | Patent family member(s) | Publication date |
|---|---|---|---|
| WO 2018187345 A1 | 11-10-2018 | AR 111204 A1 | 12-06-2019 |
| | | AR 125900 A2 | 23-08-2023 |
| | | AU 2018249817 A1 | 14-11-2019 |
| | | BR 112019020809 A2 | 28-04-2020 |
| | | CA 3058803 A1 | 11-10-2018 |
| | | CL 2019002804 A1 | 20-12-2019 |
| | | CO 2019012331 A2 | 28-02-2020 |
| | | EP 3606347 A1 | 12-02-2020 |
| | | PH 12019502199 A1 | 28-09-2020 |
| | | RU 2019131453 A | 05-05-2021 |
| | | US 2018325103 A1 | 15-11-2018 |
| | | US 2022338470 A1 | 27-10-2022 |
| | | WO 2018187345 A1 | 11-10-2018 |
| CN 109734539 B | 08-02-2022 | NONE | |
| FR 2803176 B1 | 21-11-2003 | NONE | |

Form PCT/ISA/210 (patent family annex) (April 2005)